Europäisches Patentamt

European Patent Office

Office européen des brevets

(11) Publication number: **0 094 448**
**B1**

(12) **EUROPEAN PATENT SPECIFICATION**

(45) Date of publication of patent specification: **25.06.86**

(21) Application number: **82104410.4**

(22) Date of filing: **19.05.82**

(51) Int. Cl.⁴: **A 61 L 2/06, A 23 B 9/00, A 23 L 3/18, A 23 L 3/22, A 23 P 1/00**

(54) **Method and apparatus for heat-treating powdered, granular and like materials.**

(43) Date of publication of application:
**23.11.83 Bulletin 83/47**

(45) Publication of the grant of the patent:
**25.06.86 Bulletin 86/26**

(84) Designated Contracting States:
**DE FR GB NL**

(56) References cited:
**AU-B- 441 069**
**FR-A-2 240 041**
**US-A-1 329 786**
**US-A-3 293 771**
**US-A-3 721 527**
**US-A-3 886 856**

(73) Proprietor: **KIKKOMAN CORPORATION**
**339 Noda**
**Noda-shi Chiba (JP)**

(72) Inventor: **Akao, Takeshi**
**177-1, Wakamatsu**
**Abiko-Shi Chiba (JP)**
Inventor: **Furukawa, Toshio**
**65-1, Yanagisawa**
**Noda-Shi Chiba (JP)**
Inventor: **Yamanaka, Yoshiro**
**1481-1, Yamasaki**
**Noda-Shi Chiba (JP)**
Inventor: **Okamoto, Yoshiharu**
**1871-4, Nagareyama**
**Nagareyama-shi Chiba (JP)**

(74) Representative: **Reichel, Wolfgang, Dipl.-Ing.
et al**
**Reichel und Reichel Parkstrasse 13**
**D-6000 Frankfurt am Main 1 (DE)**

EP 0 094 448 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## BACKGROUND OF THE INVENTION

### (1) Field of the Invention

This invention relates to a method and apparatus for heat or other free-flowing powdered, granular denaturation of materials such as cereals, foods and cosmetics, with a saturated or superheated steam.

### (2) Description of the Relevant Art

The present applicant has previously filed an application on "Method of Producing Soy or Bean Paste" (see Japanese Patent Publication No. 43159/1974) in which cereals are heat-treated using superheated steam and also filed an application on "Apparatus for Producing Expanded Foods" (see Japanese Patent Publication No. 26695/1970) in which cereals are also heat-treated while being allowed to flow by using superheated steam, and obtained patents on those applications.

However, both such conventional apparatus proved to involve problems in their industrial application. That is, in the former apparatus, since the raw material to be treated is transferred at high speed in an airborne heating tube, its residence time in the tube is short, and in the case of treating a large-grained material such as corn, the airborne heating tube must be made long, thus resulting in an increase in size of the treating apparatus. Besides, a device for separating the product and superheated steam, such as a cyclone, was required. The latter apparatus is also disadvantageous in that when the raw material to be treated is of a small particle size, it is blown up from within a fluidized bed and carried away by an air current. Consequently, the velocity of air current or the quantity of heat is limited and there was a limitation on the amount of treatment.

As a solution of the problem of a short heating time in the former apparatus and that of a limited heat quantity in the latter apparatus, it was considered to make the temperature of superheated steam higher, but a too high temperature thereof would badly affect the quality of product, so there was inevitably a limitation.

Both such conventional apparatus have further the disadvantage that the superheated steam must be circulated and this circulation requires a wind pressure of not lower than 19.6kPa (2,000 mmAq), thus resulting in increased power of a circulating blower and an increase in the cost of the treating apparatus.

## SUMMARY OF THE INVENTION

Having made extensive studies in view of the above-mentioned circumstances, the present inventors accomplished this invention on the basis of the finding that if a raw material fed for treatment into a pressure vessel equipped with an agitator is urged against the inner wall of the vessel by rotation of the agitator to allow it to form an annular layer along the inner wall and heat-treated directly or indirectly or in combination of both while being transferred and turned over up and down by the agitator, the period of time the raw material remains in the apparatus can be set to any desired value and the contact between the heating medium such as superheated or saturated steam and the raw material is attained to a satisfactory extent, thus permitting an effective heat treatment of the raw material.

That is, according to the present invention there are provided method and apparatus for heat-treating powdered, granular and other free-flowing materials characterized in that a raw material fed into a horizontally disposed cylindrical pressure vessel equipped with an agitator is heated while allowing it to form an annular layer along the inner wall of the pressure vessel by rotation of the agitator.

## BRIEF DESCRIPTION OF THE DRAWINGS

Fig. 1 is a front section of an apparatus according to an embodiment of the present invention and also illustrates associated devices;

Fig. 2 is a view taken along line 2—2 of Fig. 1;

Fig. 3 illustrates a centrifugal force induced by an agitator;

Fig. 4 is a front view of the apparatus partly in section;

Fig. 5 is a front view of the apparatus partly in section to which has been attached an indirect heating means;

Fig. 6(a) and (b) illustrate other indirect heating means;

Fig. 7 illustrates an embodiment of a raw material transfer adjusting device;

Fig. 8 is a sectional view taken along line 8—8 of Fig. 7;

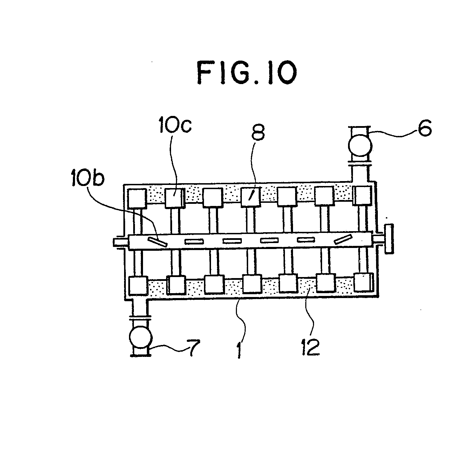

Fig. 9 is a sectional view taken along line 9—9 of Fig. 7; and

Fig. 10 illustrates another embodiment of a raw material transfer adjusting device.

## DESCRIPTION OF PREFERRED EMBODIMENTS

The present invention will be described concretely hereinunder.

Raw materials to be used in the invention are not particularly limited, for example, there may be used food raw materials such as cereals and their powdered, granulated or other free-flowing materials, small pieces of vegetables or bread crumbs, starch powder, pepper and curry powder, chemicals or chemical raw materials and their extenders, as well as foods and cosmetic raw materials.

In charging a raw material as exemplified above into a pressure vessel for heat treatment, superheated or saturated steam may be used as a direct heating medium which is brought into direct contact with the raw material, though superheated steam is preferable because the raw material can be handled in a dry condition. For raw materials which do not lose fluidity even after having absorbed a small amount of moisture, for example, cereals such as soybeans and wheat, the use of saturated steam is also effective.

When a raw material, particularly cereals, is to be denatured, in the case of treatment with saturated steam it is directly contacted with the steam at a pressure of 2 to 10 kg/cm² (gauge pressure), preferably 4 to 8 kg/cm² (gauge pressure), while in the case of treatment with superheated steam it is directly contacted with the steam at a pressure of 2 to 10 kg/cm² (gauge pressure) and at a temperature not higher than 350°C, prefereably at a pressure of 4 to 8 kg/cm² (gauge pressure) and at a temperature not higher than 320°C, for 10 seconds to 10 minutes.

As to the condition for an indirect heating, the temperature of an indirect heating medium mainly affects the treatment and too high a temperature thereof affects the quality of the product. Preferably, though depending on raw materials, the temperature of such heating medium is not higher than 350°C.

As the indirect heating medium there may be used saturated steam, superheated steam, high-temperature air, or high-temperature water, but when a heat transfer condition is considered, saturated steam is particularly preferred.

If the raw material is to be expanded, it is preferable that the pressure within the pressure vessel be as high as possible, and in this case the raw material may be suddenly discharged from the vessel into a lower pressure zone.

For carrying out the heat treatment under the conditions described above, for example, such apparatus as shown in the accompanying drawings are effective and therefore the present invention will be described more in detail hereinunder with reference to those drawings.

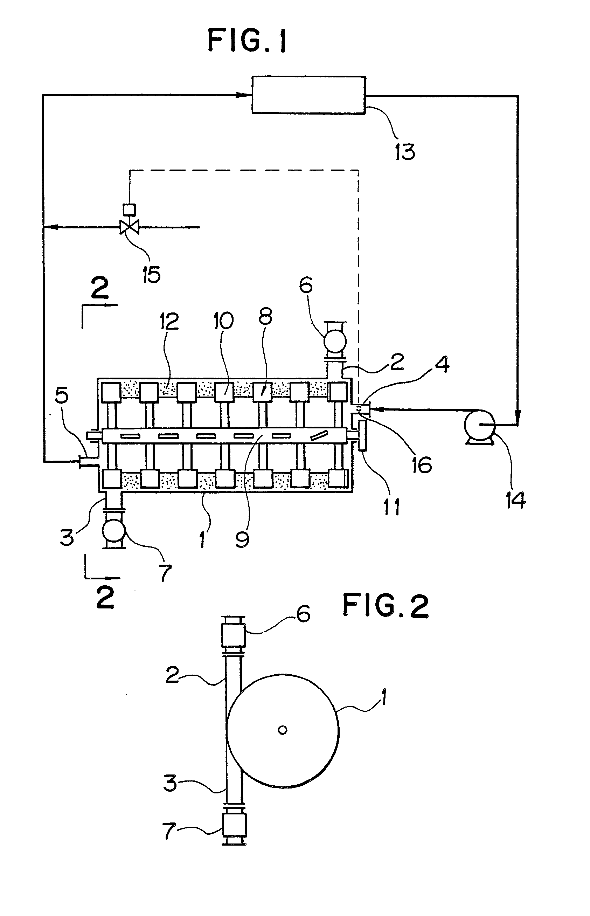

Referring first to Fig. 1, there is shown a treating apparatus according to an embodiment of the present invention wherein superheated steam is used as a direct heating medium. In Fig. 1, the reference numeral 1 designates a horizontally disposed cylindrical pressure vessel provided at one end thereof with a raw material feed port 2 and at the other end thereof with a product discharge port 3, further including a superheated steam inlet 4 and its outlet 5.

Numeral 6 designates a raw material feeding device disposed in communication with the raw material feed port 2. For example, the "Transfer Apparatus having a Forced Discharge Device" (see Japanese Patent Publication No. 8927/1970) filed by the present applicant may be utilized as the raw material feeding device 6. Numeral 7 designates a product discharge device of the same structure as the raw material feeding device 6, and it communicates with the product discharge port 3.

Numeral 8 designates an agitator mounted within the pressure vessel 1, and it is constituted of a rotatable shaft 9 coaxially inserted in the pressure vessel 1 and a plurality of blades 10, the blades 10 being radially fixed to the shaft 9 and having a diameter of rotational locus at their tip ends approximately equal to the inside diameter of the pressure vessel 1. The blades 10 are inclined in parallel or spirally with respect to the axial direction of the shafft 9 and are constructed so as to transfer a raw material to be treated from the raw material feed port 2 side to the product discharge port 3 side while turning it over up and down.

Numeral 11 designates a pulley attached to an end portion of the shaft 9. The agitator 8 is driven by a motor (not shown) or the like through the pulley 11.

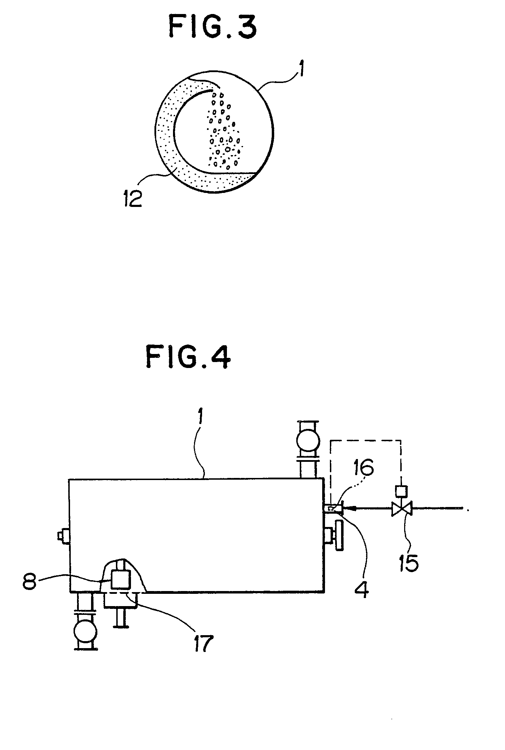

Numeral 12 designates a raw material fed into the pressure vessel 1 by means of the raw material feeding device 6. The raw material 6 is given a centrifugal force by virtue of rotation of the agitator 8 and forms an annular layer as shown in Fig. 1. In this case, the minimum number of revolutions of the agitator required to form such an annular layer will be referred to hereinafter as the centrifugal revolution. Thus, the agitator 8 is required to impart to the raw material 12 a centrifugal force sufficient to form such an annular layer. For example, as shown in Fig. 3, if the raw material 12 drops freely from near the upper part of the pressure vessel 1, there will occur variations in residence time of the raw materials in the vessel 1, and therefore such a phenomenon is not preferable.

When conditions necessary for the raw material 12 to form an annular layer are considered, the centrifugal revolution N (in rpm) of the agitator 8 theoretically becomes as follows:

$$N = \sqrt{g \cdot r} \cdot \frac{60}{2\pi \cdot r}$$

g: gravitational acceleration (m/s²)
r: radius of gyration of the raw material (m).

However, since there is a deviation between the velocity of the tip end portion of the blades 10 and that of the raw material, it is necessary to make the actual centrifugal revolution larger than the aforesaid theoretical value.

It is desirable that the raw material feed port 2 and the product discharge port 3 be formed tangentially with respect to the pressure vessel 1. For example, in case the agitator 8 rotates in a counterclockwise direction in Fig. 2, if the raw material feed port 2 and the product discharge port 3 are disposed as shown, the feed of raw material and the discharge of product will be effected smoothly.

The residence time of the raw material 12 in the pressure vessel 1 can be adjusted by changing the number of revolutions of the agitator 8 or the inclination of the blades 10.

Numeral 13 designates a superheater for re-heating superheated steam whose temperature has been

3

lowered after heating the raw material 12. Numeral 14 designates a circulating blower for circulating superheated steam for the purpose of its re-utilization. Furthermore, numeral 15 designates a control valve communicating with a source of saturated steam such as a boiler or the like. The control valve 15 operates according to a signal from a pressure detector 16 mounted in the interior of the pressure vessel 1 to maintain the pressure within the system at a predetermined level.

According to this embodiment constructed as above, the raw material 12 once fed into the pressure vessel 1 by means of the raw material feeding device 6 is immediately given a centrifugal force by the agitator 8 and thereby urged in the form of a layer against the inner wall of the vessel 1. Then, the raw material 12 is transferred toward the product discharge port 3 while being turned over up and down and forming an annular layer. The raw material 12, during its transfer, is denatured with superheated steam and is discharged to the exterior through the discharge port 3 and then through the product discharge device 7.

The following description is now provided for the case where the direct heating medium is saturated steam, with reference to Fig. 4.

In the embodiment shown in Fig. 4, the heating medium inlet 4 communicates through the control valve 15 with a source of saturated steam such as a boiler, and in the same way as in the embodiment of Fig. 1 the pressure detector 16 is mounted in the interior of the pressure vessel 1 and with a signal from the detector 16 the control valve 15 is controlled to maintain the pressure within the vessel 1 at a predetermined level.

On the other hand, the condensate of the saturated steam may be drained through a perforated plate 17 mounted at a lower end portion of the pressure vessel 1 as showwn in the figure. But such a drainage is not always required when treating cereals or the like because in the treatment of these materials the condensate is absorbed therein.

In case a raw cereal material (containing 10% W/W moisture) which has been preheated to 50°C is to be treated with saturated steam at a pressure of 6 kg/cm$^2$ (gauge pressure), even if the condensate of the steam is wholly absorbed by the raw material, the water content of the raw material becomes only about 17% W/W. Such a small amount of moisture does not affect the fluidity of the raw material and it is possible to handle the raw material in just the same manner as when superheated steam is used.

In the apparatus of the present invention, moreover, cereals or the like, up to a water content of 30% W/W, do not adhere to the agitator 8 or the inner wall of the pressure vessel 1, so even if the addition of water or like treatment is applied to the raw material before treatment, the raw material is fully capable of being treated according to the present invention.

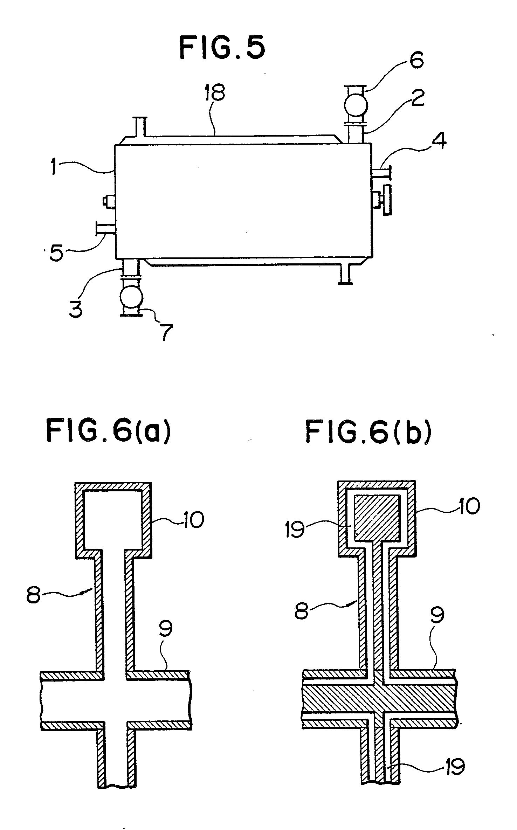

Referring now to Fig. 5, there is shown another embodiment of the present invention, wherein a jacket 18 as an indirect heating means is mounted throughout the outer periphery of the pressure vessel 1 shown in the embodiment of Fig. 1.

In the embodiment shown in Fig. 5, an indirect heating medium such as saturated steam, superheated steam, high-temperature air or high-temperature water is passed through the jacket 18, whereby the raw material 12 fed into the pressure vessel 1 is indirectly heated, or the pressure vessel 1 can be kept warm.

In this embodiment constructed as above, the heat load of directly heating the raw material within the pressure vessel 1 can be decreased, so the circulating blower can be made smaller in size in case the heating is carried out with superheated steam. Furthermore, because the amount of a superheated steam flowing is decreased, even when finely divided particles of the raw material 12 float within the pressure vessel 1, those particles will not be carried away by the steam.

On the other hand, in case the direct heating medium is saturated steam, the amount of its condensate can be decreased and therefore the raw material 12 caan be treated in a more highly dried condition.

In the present invention, moreover, the raw material 12 forms an annular layer throughout the inner periphery of the pressure vessel 1, therefore the method of indirectly heating the raw material from throughout the outer peripheral wall of the vessel 1 as in this embodiment is particularly effective.

As another method for indirectly heating the raw material 12, the blade 10 may be constructed as a hollow blade as shown in Fig. 6(a) and saturated steam may be introduced into the hollow portion, or a passage 19 for circulation of an indirect heating medium may be formed in the agitator 8 as shown in Fig. 6(b).

Known means may be adopted for introducing steam into the hollow portion or the passage 19 in the agitator 8.

Even with only the indirect heating method as in this embodiment without directly introducing a heating medium into the pressure vessel 1, the raw material 12 can be heat-treated, but if water is added to the raw material 12 in advance, the pressure within the pressure vessel 1 will be stabilized and more desirable results will be obtained.

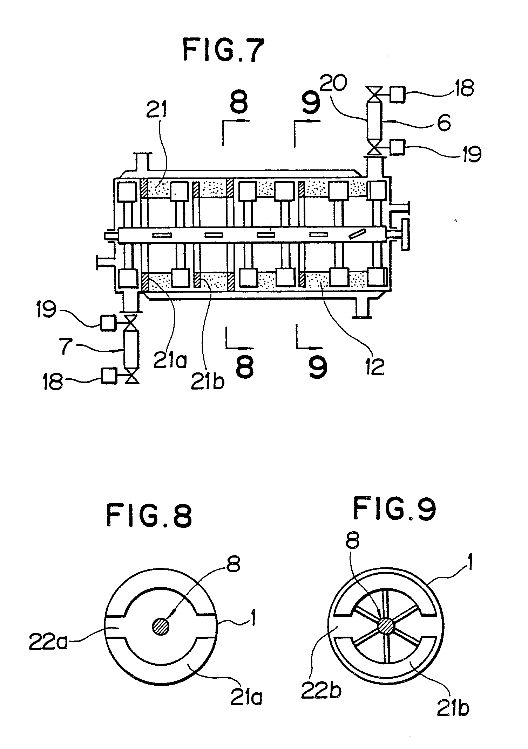

Referring now to Fig. 7, there is shown another embodiment, in which a raw material transfer adjusting device is mounted within the pressure vessel 1. This adjusting device comprises a ring-like weir 21 having a notch, and it is may be changed according to the residence time of the raw material 12 or the height of its layer or for further stabilization. The weir 21 may be mounted so as to dam up the flow of the raw material 12. For example, it may be a weir 21a mounted on the inner wall of the pressure vessel 1 or a weir 21b fixed to the agitator 8 and having an inside diameter approximately equal to that of the vessel 1.

The raw material 12 moves through notched portions 22a and 22b of the weirs 21a and 21b, respectively while being restricted by the height of the weirs 21a and 21b, and its residence time in the

4

pressure vessel 1 becomes stable. If a large number of weirs 21 are disposed, the height and residence time of the raw material 12 will become more stable, thereby permitting a more uniform execution of the heat treatment. Moreover, if the weir 21 is mounted removably with bolts or the like, it becomes possible to easily change the height of the weir 21, the number of notched portion and that of the weir to be mounted, whereby the treating apparatus of the invention becomes applicable to a wider variety of raw materials.

In this embodiment, a pair of automatic valves 18 and 19 are used as the raw material feeding device 6 and also as the product discharge device 7. That is, the devices 6 and 7 are constructed by letting the automatic valves 18 and 19 communicate with each other through a connecting pipe 20, and by opening and closing these valves alternately the introduction of raw material and the discharge of product are effected while keeping the pressure vessel 1 airtight.

In place of the raw material transfer adjusting device or the weir 21, blades 10b and 10c on the product discharge side of the agitator 8 may be constructed so as to have inclinations which allow the raw material 12 to be returned to its feed side. In this case, by changing the inclination of the blades it is possible to adjust the height of the layer of the raw material 12 or its residence time.

Because the apparatus of the present invention is constructed as above, it allows more freedom with respect to its operating conditions such as the pressure and temperature of a direct heating medium and of an indirect heating medium as well as the amount of those heating media and the residence time of a raw material in the pressure vessel, and consequently various raw materials can be treated regardless of their kind and particle size.

Furthermore, in the case of using and circulating superheated steam as a direct heating medium, a raw material will not be carried away by the steam despite its direct contact therewith, thus eliminating the need to use a cyclone or the like for separating the two, and if an indirect heating means is used in combination with the direct heating means, as shown in Fig. 5, it will become less possible that the raw material will be carried away by the steam, because in this case the heat load of the direct heating means can be decreased.

In the present invention, moreover, the heat transfer area of a raw material and that of the indirect heating means are so large that the heat of the indirect heating means can be utilized effectively, and since the raw material is transferred while being turned over up and down by means of the agitator, its contact with the direct heating medium can be made effectively.

Working examples of the present invention will be described hereinunder to further illustrate the invention, in which working examples the size of the pressure vessel used was 0.25 m in diameter, the length of the raw material feed port and that of the product discharge port were each 2.7 m and the heat transfer area of the jacket was 2.0 m².

## Example 1

Wheat (water content: 11% W/W) was fed at a rate of 330 kg. per hour into a pressure vessel in which an agitator was rotating at 250 rpm and in which had been introduced superheated steam having a pressure of 6 kg/cm² (gauge pressure) and a temperature of 250°C, while saturated steam having a pressure of 12 kg/cm² (gauge pressure) was allowed to flow through a jacket. After heating in the vessel for 120 seconds in the presence of the superheated steam, the raw material was discharged continuously into the atmosphere to obtain a denatured wheat having a water content of 5% W/W and an alphalization degree (see Note 1) of 75%. At this time, the temperature of the superheated steam at the outlet of the pressure vessel was 170°C, and the superheated steam was used at a rate of 350 kg. per hour.

## Example 2

Corn (water content: 10.5% W/W) was fed at a rate of 250 kg. per hour into a pressure vessel in which an agitator was rotating at 250 rpm and in which had been introduced superheated steam having a pressure of 6 kg/cm² (gauge pressure) and a temperature of 300°C, while saturated steam having a pressure of 7 kg/cm² (gauge pressure) was allowed to flow through a jacket. After heating in the vessel for 140 seconds in the presence of the superheated steam, the raw material was discharged continuously into the atmosphere to obtain a denatured wheat having a water content of 6% W/W and an alphalization degree of 72%. At this time, the temperature of the superheated steam at the outlet of the pressure vessel was 210°C, and the superheated steam was used at a rate of 500 kg. per hour.

In this Example, the flowing of the saturated steam through the jacket was for the purpose of keeping warm.

## Example 3

Crushed soybean (particle size: 12—16 mesh (1.0—1.41 mm), water content: 12% W/W) was fed at a rate of 1,500 kg. per hour into a pressure vessel in which an agitator was rotating at 350 rpm and in which had been introduced saturated steam having a pressure of 6 kg/cm² (gauge pressure), while saturated steam having a pressure of 7 kg/cm² (gauge pressure) was allowed to let flow through a jacket. After, heating in the vessel for 30 seconds in the presence of the saturated steam, the raw material was discharged continuously into the atmosphere to obtain a denatured soybean having a water content of 14% W/W and a digestibility (see Note 2) of 95%. In this operation the saturated steam was used at a rate of 300 kg. per hour. The flowing of the saturated steam through the jacket in this Example was for the purpose of keeping warm.

## Example 4

Defatted soybean meal (water content: 12% W/W) was fed at a rate of 220 kg per hour into a pressure vessel in which an agitator was rotating at 250 rpm and in which had been introduced saturated steam having a pressure of 6 kg/cm² (gauge pressure), while saturated steam having a pressure of 12 kg/cm² (gauge pressure) was allowed to flow through a jacket. After heating in the vessel for 100 seconds in the presence of the saturated steam, the raw material was discharged continuously into the atmosphere to obtain a denaturated soybean having a water content of 7% W/W/ and a digestibility of 95%. In this operation the saturated steam was used at a rate of 200 kg per hour.

## Example 5

14 kg of wheat (water content: 14% W/W) was fed into a pressure vessel in which an agitator was rotating at 250 rpm. On the other hand, saturated steam having a pressure of 12 kg/cm² (gauge pressure) was allowed to flow through a jacket to heat the raw material indirectly, while the interior of the pressure vessel was held at 6 kg/cm² (gauge pressure). After heating for 6 minutes, the raw material was discharged into the atmosphere to obtain a denaturated wheat having a water content of 9.4% W/W and an alphalization degree of 73%.

### Note 1
### Alphalization Degree

A sample passing a 32 mesh sieve (0.5 mm) prepared from analysis of the raw material is placed in an amount of 500 mg into each of two Erlenmeyer flasks each having a capacity of 150 ml, then 40 ml of water is added into each of the flasks and the contents of the flasks are stirred thoroughly. One is made a measuring zone, into which is added 20 ml of a measuring buffer solution, while the other is made a completely alphalized zone, into which are added 5 ml of 2N·NaOH and then 16 ml of 1M acetic acid.

5 ml of an enzyme solution is added to each of the test solutions in a thermostatic vessel held at 37°C to allow reaction to take place. After 60 minutes, 4 ml of 2N·NaOH is added and the reaction is stopped. Then, the reaction product is charged into a 100 ml graduated measuring flask to a constant volume and filtered through a No. 5A filter paper. With respect to 8 ml of the filtrate, the resulting sugar is determined according to the SOMOGYI method. The result is expressed in percentage by the following equation:

$$\text{Alphalization} = \frac{\text{Amount of sugar in the measuring zone}}{\text{Amount of sugar in the completely alphalized zone}} \times 100(\%)$$

Measuring buffer solution:
2N·NaOH and 1M acetic acid are mixed at a ratio of the former to the latter of 5:16.

Enzyme solution:
0.6 g of matsulase · M-00 (manufactured by Matsutani Chemical Co.) is placed in a 200 ml. beaker, then about 150 ml of water and 5 ml of a 0.4M acetic acid buffer solution are added, followed by stirring for 30 minutes with a stirrer The mixture is weighed to a constant volume of 250 ml and then filtered through a No. 5A filter paper.

### Note 2
### Digestibility

For the measurement of digestibility, the denatured soybean after heat treatment is dried at a low temperature under reduced pressure and then pulverized, thereafter 1 g of the resulting powder is placed in a shaking type test tube, and after adding 10 ml of a 0.5 mol phosphoric acid buffer solution (pH 7.2), 20 ml of an enzyme liquid (see Note below) and 1 ml of triol, the test tube is stoppered. Zymolysis is allowed to take place for 7 days at 37°C while shaking the test tube slowly, then distilled water is added to the zymolyzate to a total volume of 100 ml followed by separation into liquid and solid phases by centrifugal separation. 15 ml of 1.2 mol trichloroacetic acid is added to 30 ml of the liquid phase portion, then the resulting precipitate (undecomposed protein) is filtered off and 5 ml of the filtrate is sampled and measured for its nitrogen content according to the Kje dahl method. Separately, without adding the foregoing powder, a blank test is made in the same procedure as above, and a value resulting from subtraction of the latter value from the former value is assumed to be A. On the other hand, the nitrogen content in one gram of the powder sample is determined by the Kje dahl method and the value obtained is assumed to be B, then digestibility is calculated from the following equation:

$$\text{Digestibility (\%)} = \frac{A \times \dfrac{30 + 15}{5} \times \dfrac{100}{30}}{B} \times 100 = \frac{A \times 30}{B} \times 100$$

Note) The aforesaid enzyme liquid indicates an extract having a proteinase activity of

$$53 \text{ (PU)} \frac{\text{cas·}30°\text{·FR·}}{\delta - \text{tyr.}} / \text{ml}$$

extracted from bran enzyme of aspergillus sojae which is a typical koji mould used in soy brewing. In connection with the above proteinase activity,

$$1\text{(Pu)} \frac{\text{cas·}30°\text{·FR·}}{\delta - \text{tyr.}} / \text{ml}$$

means an enzymatic activity presenting Folin color in an amount corresponding to $1\delta$ of tyrosine per minute when enzymatic reaction is carried out at pH 7.2 and at 30°C using 1% milk casein as a substrate.

**Claims**

1. A method for denaturing a powdered, granular or material as a raw material, characterized by feeding the raw material into a horizontally installed cylindrical pressure vessel equipped with an agitator and heating the raw material directly, indirectly or directly and indirectly with a heating medium while allowing an annular layer of the raw material to be formed along an inner wall of said pressure vessel by means of rotation of said agitator, said direct heating being performed with superheated steam or a saturated steam at a pressure of 2 to 10 kg/cm$^2$.

2. The heat-treating method of claim 1, characterized in that the heating medium for indirect heating is superheated steam, saturated steam, high-temperature air or high-temperature water.

3. An apparatus for denaturing a powdered, granular or material, comprising:

a horizontally installed cylindrical pressure vessel (1) provided at one end thereof with a raw material feed port (2) and at the other end thereof with a product discharge port (3), further having an indirect heating means (18) and an inlet port (4) for a heating medium and constructed so that said heating medium can be introduced into the interior thereof;

a material feeding device (6) communicating with said raw material feed port (2);

a product discharge device (7) communicating with said product discharge port (3);

an agitator (8) mounted within said pressure vessel, said agitator being rotated at least at a centrifugal revolution; and

a means (21) for adjusting the residence time of the raw material in the pressure vessel mounted within said pressure vessel.

4. The heat-treating apparatus of claim 3, wherein said agitator (8) comprises a rotatable shaft (9) inserted coaxially into said pressure vessel (1) and a plurality of blades (10) fixed radially to said shaft, the diameter of a rotational locus at tip ends of said blades being nearly equal to the inside diameter of said pressure vessel, said blades being inclined in parallel or spirally with respect to the axial direction of said shaft to transfer the raw material (12) from said raw material feed port (2) to said product discharge port (3), said adjusting means comprises the inclination of the blades.

5. The heat-treating apparatus of claim 3, wherein said adjusting means (21) comprises a ringlike weir (21a) fixed to an inner wall of said pressure vessel (1) at nearly a right angle to the longitudinal direction of said pressure vessel, said weir having at least one notched portion (22a).

6. The heat-treating apparatus of claim 3, wherein said adjusting device (21) comprises a ringlike weir (21b) having a diameter nearly equal to the inside diameter of said pressure vessel (1) and fixed to said shaft (9), said weir having at least one notched portion (22b).

7. The heat-treating apparatus of claim 3, wherein said indirect heating means (18) comprises a jacket mounted on the outer periphery of said pressure vessel (1).

8. The denaturing method of claim 1, wherein the direct heating is performed at a temperature not higher than 350°C.

9. The denaturing method of claim 1, wherein the direct heating is performed for 10 seconds to 10 minutes.

7

## 0 094 448

**Patentansprüche**

1. Verfahren zum Denaturieren eines pulvrigen oder körnigen Gutes in Form eines Rohmaterials, dadurch gekennzeichnet, daß man mit dem Rohmaterial ein waagerecht angeordnetes, mit einem Rührer ausgestattetes, zylindrisches Druckgefäß beschickt und das Rohmaterial mit einem Heizmittel direkt, indirekt, oder direkt und indirekt erhitzt, während man eine ringförmige Schicht des Rohmaterials sich längs der Innenwand des Druckgefäßes durch Rotieren lassen des Rührers bilden läßt, wobei das direkte Erhitzen mit überhitztem oder gesättigtem Wasserdampf bei einem Druck von 2 bis 10 kg/cm² erfolgt.

2. Wärmebehandlungsverfahren gemäß Anspruch 1, dadurch gekennzeichnet, daß man als Heizmittel für das indirekte Erhitzen überhitzten Wasserdampf, gesättigten Wasserdampf, Luft von hoher Temperatur oder Wasser von hoher Temperatur verwendet.

3. Vorrichtung zum Denaturieren eines pulverigen oder körnigen Gutes, bestehend aus einem waagerecht angeordneten, zylindrischen Druckgefäß (1), das an seinem einem Ende mit einer Beschickungsöffnung (2) für Rohmaterial und an seinem anderen Ende mit einer Entleerungsöffnung (3) für das Produkt ausgestattet ist, das weiter ein indirektes Heizmittel (18) sowie einen Einlaß (4) für ein Heizmittel aufweist und derart aufgebaut ist, daß das Heizmittel in sein Inneres eingeleitet werden kann, einer Materialbeschickungeinrichtung (6), die mit der Beschickkungsöffnung (2) für das Rohmaterial in Verbindung steht, eine Produktausbringungseinrichtung (7), die mit dem Produktauslaß (3) in Verbindung steht, einem Rührer (8), der innerhalb des Druckgefäßes angeordnet ist und mindestens mit einer Zentrifugalkräfte verleihenden Rotationsgeschwindigkeit betrieben wird, und einem innerhalb des Druckgefäßes angeordneten Mittel (21) zum Einstellen der Verweilzeit des Rohmaterials in dem Druckgefäß.

4. Wärmebehandlungsvorrichtung gemäß Anspruch 3, dadurch gekennzeichnet, daß der Rührer (8) aus einer drehbaren Welle (9), die koaxial in das Druckgefäß (1) eingesetzt ist, sowie einer Anzahl von radial an der Welle befestigten Armen (10) besteht, wobei der Durchmesser des von einem rotierenden Punkt an den Endspitzen der Arme beschriebenen Kreises nahezu dem Innendurchmesser des Druckgefäßes gleich ist, die Arme bezüglich der axialen Richtung der Welle parallel oder spiralförmig geneigt sind, um das Rohmaterial (12) von dem Rohmaterialbeschickungseinlaß (2) zum Produktauslaß (3) zu befördern, und die Einstellmittel die Neigung der Arme darstellen.

5. Wärmebehandlungsvorrichtung gemäß Anspruch 3, dadurch gekennzeichnet, daß die Einstellmittel (21) aus einem ringförmigen Wehr (21a) bestehen, das in nahezu rechtem Winkel zur Längsrichtung des Druckgefäßes an der Innenwand des Druckgefäßes (1) befestigt ist und das mindestens eine Ausnehmung (22a) aufweist.

6. Wärmebehandlungsvorrichtung gemäß Anspruch 3, dadurch gekennzeichnet, daß das Einstellmittel (21) aus einem ringförmigen Wehr (21b) besteht, das einen Durchmesser aufweist, der nahezu gleich dem Innendurchmesser des Druckgefäßes (1) ist, und das an der Welle (9) befestigt ist und mindestens eine Ausnehmung (22b) aufweist.

7. Wärmebehandlungsvorrichtung gemäß Anspruch 3, dadurch gekennzeichnet, daß das indirekte Heizmittel (18) aus einem Mantel besteht, der an dem äußeren Umfang des Druckgefäßes (1) montiert ist.

8. Denaturierungsverfahren gemäß Anspruch 1, dadurch gekennzeichnet, daß das direkte Erhitzen bei einer Temperatur von nicht über 350°C durchgeführt wird.

9. Denaturierungsverfahren gemäß Anspruch 1, dadurch gekennzeichnet, daß das direkte Erhitzen während 10 Sekunden bis 10 Minuten durchgeführt wird.

**Revendications**

1. Procédé de denaturation d'une matière pulvérulente, granulaire ou autre matière ruisselable en tant que matière brute, caractérisé par l'alimentation de la matière brute dans un récipient sous pression cylindrique, horizontal, équipé d'un agitateur, et le chauffage de la matière brute directement, indirectement ou à la fois directement et indirectement par un milieu chauffant, tout en permettant la formation d'une couche annulaire de la matière brute le long d'une paroi interne de ce recipient sous pression, grâce à la rotation de l'agitateur susdit, le chauffage direct étant réalisé grâce à une vapeur surchauffée ou à une vapeur saturée à une pression de 2 à 10 kg/cm².

2. Procédé de traitement thermique suivant la revendication 1, caractérisé en ce que le milieu chauffant pour le chauffage indirect est constitué par une vapeur surchauffée, une vapeur saturée, de l'air à haute température ou de l'eau à haute température.

3. Appareil de dénaturation d'une matière pulvérulente, granulaire ou autre matière ruisselable, comprenant:
un récipient sous pression cylindrique horizontal (1) comportant, à une extrémité, une lumière d'alimentation de matière brute (2) et, à l'autre extrémité, une lumière de décharge de produit (3), ce récipient comportant en outre un dispositif de chauffage indirect (18) et une lumière d'entrée (4) pour un milieu chauffant, ce récipient étant construit de telle sorte que ce milieu chauffant puisse être introduit à l'intérieur de ce récipient;

8

# 0 094 448

un dispositif d'alimentation de matière (6) communiquant avec la lumière d'alimentation de matière brute (2);

un dispositif de décharge de produit (7) communiquant avec la lumière susdite de décharge de produit (3);

un agitateur (8) monté à l'intérieur du récipient sous pression, cet agitateur étant mis en rotation au moins à une révolution centrifuge; et

un dispositif (21) prévu pour régler la durée de séjour de la matière brute dans le récipient sous pression et monté à l'intérieur de celui-ci.

4. Appareil de traitement thermique suivant la revendication 3, caractérisé en ce que l'agitateur (8) comprend un arbre rotatif (9) monté coaxialement à l'intérieur du récipient sous pression (1) et un série de palettes (10) fixées radialement sur cet arbre, le diamètre de rotation des extrémités extérieures des palettes étant presque égal au diamètre interne du récipient sous pression, ces palettes étant inclinées parallèlement ou en spirale par rapport à la direction axiale de l'arbre afin de transférer la matière brute (12) depuis la lumière d'alimentation de matière brute (2) vers la lumière de décharge de produit (3), le dispositif de réglage susdit englobant l'inclinaison des palettes.

5. Appareil de traitement thermique suivant la revendication 3, caractérisé en ce que le dispositif de réglage (21) comprend un déversoir annulaire (21a) fixé à la paroi interne du récipient sous pression (1) pratiquement perpendiculairement à la direction longitudinale du récipient sous pression, ce déversoir comportant au moins une partie entaillée (22a).

6. Appareil de traitement thermique suivant la revendication 3, caractérisé en ce que le dispositif de réglage (21) comprend un déversoir annulaire (21b) d'un diamètre presque égal au diamètre interne du récipient sous pression (1) et fixé à l'arbre (9) susdit, ce déversoir comportant au moins une partie entaillée (22b).

7. Appareil de traitement thermique suivant la revendication 3, caractérisé en ce que le dispositif de chauffage indirect (18) consiste en une chemise montée sur la périphérie externe du récipient sous pression (1).

8. Procédé de dénaturation suivant la revendication 1, caractérisé en ce que le chauffage direct est réalisé à une température non supérieure à 350°C.

9. Procédé de dénaturation suivant la revendication 1, caractérisé en ce que le chauffage direct est réalisé pendant 10 secondes à 10 minutes.

9

0 094 448

# FIG.1

# FIG.2

1

## FIG.3

## FIG.4

## FIG.5

## FIG.6(a)

## FIG.6(b)

# FIG.7

# FIG.8

# FIG.9

# FIG.10